# EUROPÄISCHE PATENTSCHRIFT

(11) **EP 2 065 702 B1**
(45) Veröffentlichungstag und Bekanntmachung des Hinweises auf die Patenterteilung: **08.02.2012**
(21) Anmeldenummer: 09002448.0
(22) Anmeldetag: 10.10.2002
(51) Int. Cl.: G01N 30/00, G01N 33/53, C12N 15/10, B01J 19/00, C12Q 1/68, B01L 3/00

(54) **Mikrofluidisches Extraktionsverfahren**
Microfluid extraction method
Procédé d'extraction microfluidique

(30) Priorität: 10.10.2001 DE 10149947
(43) Veröffentlichungstag der Anmeldung: 03.06.2009
(62) Teilanmeldung aus: 02781236.1
(73) Patentinhaber: febit holding GmbH, 69120 Heidelberg (DE)
(72) Erfinder: Müller, Manfred, 79541 Lörrach (DE); Stähler, Cord F., 69493 Hirschberg a.d. Bergstraße/Großsachsen (DE); Stähler, Peer F., 68167 Mannheim (DE)
(74) Vertreter: Weiss, Wolfgang

(56) Entgegenhaltungen:
- EP-A- 0 846 776
- WO-A-01/40509
- WO-A-02/12855
- OKANO K ET AL: "Position-specific release of DNA from a chip by using photothermal denaturation" SENSORS AND ACTUATORS B, ELSEVIER SEQUOIA S.A., LAUSANNE, CH, Bd. 64, Nr. 1-3, 1. Juni 2000 (2000-06-01), Seiten 88-94, XP004199291 ISSN: 0925-4005
- BRUIN G J M: "RECENT DEVELOPMENTS IN ELECTROKINETICALLY DRIVEN ANALYSIS ON MICROFABRICATED DEVICES" ELECTROPHORESIS, WEINHEIM, DE, Bd. 21, 2000, Seiten 3931-3951, XP001031800 ISSN: 0173-0835

## Beschreibung

Die Erfindung betrifft ein Verfahren zur gezielten parallelen Extraktion von einer Vielzahl von individuellen DNA- oder RNA-Molekülen aus einem Gemisch.

Die selektive Extraktion von Molekülen ist für viele Arbeitsgebiete in der Biochemie, Biologie und Medizin ein zentraler und wichtiger Prozess. Zu diesen Arbeitsgebieten gehört die Extraktion und Aufreinigung von Nukleinsäuren, Proteinen, Zuckern und weiteren biochemischen funktionellen Molekülen.

Auch in der Genetik spielen biochemische Methoden zur Extraktion von bestimmten Molekülen, Verbindungen oder Stoffklassen eine wichtige Rolle. Besondere Bedeutung kommt dabei der Aufreinigung von Nukleinsäuren, meist DNA und RNA, zu. Wesentliche Schritte der Rekombinationstechnik setzen die Isolation und Reinigung von bestimmten Nukleinsäuren, z.B. von Plasmid-DNA, voraus. Auch die Konstruktion von Gen-Bibliotheken aus messenger-RNA (mRNA), die eine zentrale Bedeutung in der Gentechnik hat, ist auf die Isolation einer gewünschten RNA-Population angewiesen. Aus solchen Bibliotheken werden Gene oder Genfragmente "gefischt", um sie weiter untersuchen oder manipulieren zu können.

Biochemische, biologische und medizinische Analyseverfahren lassen sich durch Miniaturisierung und Parallelisierung in ihrer Effizienz und Aussagekraft enorm steigern. Solche Miniaturisierungen betreffen z.B. die Analyse von genetischem Material mit Hilfe von Hybridisierungsexperimenten auf DNA-Mikroarrays. Durch Entwicklung von Mikroarrays aus geeigneten DNA-Sonden als Rezeptoren lassen sich ganze Genome und Transkriptome analysieren. Für die Herstellung und Anwendung der Mikroarrays spielen Extraktionsverfahren eine wichtige Rolle und sind zugleich ein wichtiger Kostenfaktor, wenn es sich um solche Mikroarrays handelt, bei der die entsprechenden DNA-Polymersonden unabhängig vom Reaktionsträger synthetisiert ("off chip" Synthese) und dann auf den Reaktionsträger aufgebracht werden. Neben den recht weit verbreiteten DNA-Mikroarrays sind auch Verfahren miniaturisiert und parallelisiert worden, die dem Screening nach Molekülen mit besonderen Eigenschaften dienen, z.B. Ribozymen. Weitere Beispiele für Rezeptoren auf Mikroarrays sind Proteine und solche Moleküle, die in der Natur nicht vorkommen, wie z.B. Peptidnukleinsäuren (PNA). Viele solcher Assay-Formate werden unter der Rubrik Biochips zusammengefasst. Für nahezu alle diese Assay-Formate und Biochips sind die Isolation und Reinigung des Probenmaterials zentrale Prozesse der Probenvorbereitung.

Mikroreaktionstechniken können mit solchen Mikroarrays gekoppelt werden, um sowohl bei Probenvorbereitung als auch bei Herstellung des eigentlichen Arrays zu schnellen und effizienten Systemen zu kommen. Dies schließt auch die Verwendung von mikrofluidischen Verfahren mit ein.

Für die biochemische Isolation von Nukleinsäuren stehen zahlreiche Methoden zur Verfügung. Die Methoden erlauben zwar die Isolation und gegebenenfalls auch Aufreinigung von RNA oder DNA über ihre biophysikalischen Eigenschaften, sind dabei aber völlig unspezifisch in Bezug auf die Sequenz des Nukleinsäurestranges.

So können aus Gesamt-RNA, die unterschiedliche Sorten RNA enthält, mRNA-Moleküle relativ spezifisch isoliert werden, indem man auf einer festen Phase, z.B. Latex-Beads, Magnet-Beads, Controlled Pore Glass-Beads oder der Matrix einer Säule Poly-Thymidin Stränge (poly-T Stränge) immobilisiert. Diese Poly-T Stränge hybridisieren nach Zugabe von Gesamt-RNA mit dem Poly-Adenin (poly-A) Schwanz von mRNA-Molekülen und erlauben das Abtrennen der nichtgebundenen RNA-Moleküle. Die mRNA-Moleküle werden dann isoliert, indem der Puffer entsprechend so geändert wird, dass die Hybridisierung zugunsten von Einzelsträngen aufgehoben wird. Die frei werdenden mRNA-Moleküle kann man anschließend eluieren.

Der Informationsgehalt einer solchen Isolationsmatrix ist vergleichsweise gering, da nur zwei Kategorien von Zielmolekülen unterschieden werden können, nämlich mit oder ohne poly-A Schwanz.

Im Falle von Isolationsverfahren für Proteine verhält es sich zumeist ähnlich. So werden. Immunglobuline häufig durch eine Isolationsmatrix in einer Säule mit immobilisiertem Protein A (aus Staphylococcus aureus) isoliert (Brown et al., Biochem. Soc. Transactions (England) 26 (1998), 249). In anderen Versionen des Verfahrens werden Antikörper für eines oder wenige Zielmoleküle an die Isolationsmatrix gebunden.

Solche Isolationsverfahren werden allgemein als Affinitätschromatographie bezeichnet. Ein Nachteil dieser Verfahren besteht darin, dass keine parallele selektive Isolation unterschiedlicher Zielmoleküle möglich ist.

US-Patent 6,013,440 beschreibt ein Verfahren zur Herstellung einer Affinitätsmatrix, wobei ein Satz unterschiedlicher Nukleinsäuresonden auf einem festen Träger immobilisiert wird, um auf diese Weise Zielnukleinsäuren einer noch unbekannten Sequenz aus einer Probe anzureichern. Neben anderen Nachteilen ist hervorzuheben, dass das vorgeschlagene Verfahren flächige Träger als Isolationsmatrix verwendet, die nur eine ungünstige Elution erlauben. Auch mit diesem Verfahren können somit die aus dem Stand der Technik bekannten Nachteile nicht beseitigt werden.

EP 0 846 776 offenbart eine Testvorrichtung zur Isolation eines Analyten aus einer Probe, wobei die Vorrichtung eine Röhre mit einem linearen Array von Bindeelementen offenbart, welche jeweils mit einem unterschiedlichen Bindefaktor verbunden sind, an welchen ein korrespondierender spezifischer Bestandteil der Probe bindet. Die Bindeelemente sind auf die innere Fläche der Röhre aufgebracht, so dass der Analyt in einer schmalen Zone (d.h. an einem Bindeelement) konzentriert und isoliert wird.

Okano et al. (Sensors and Actuators B 64, 2000: 88-94) offenbart eine positionsspezifische Freisetzung von DNA von einem Chip durch photothermale Denaturierung. Das Verfahren besteht aus fünf Schritten: (1) Hybridisieren einer DNA-Probe mit Sonden, welche auf dem Chip fixiert sind, (2) Entfernen unspezifisch gebundener DNA durch Waschen, (3) Erwärmen eines kleinen Bereichs auf dem Chip durch IR-Laser, um eine spezifische DNA von der Oberfläche zu lösen, (4) Sammeln der freigesetzten DNA, und (5) Wiederholen der Schritte 3 und 4, um viele DNA-Fragmente zu gewinnen, welche auf dem Chip fixiert sind.

Mit der vorliegenden Erfindung wird ein Verfahren zur Verfügung gestellt, bei denen ein mikrofluidischer Träger als Isolationsmatrix verwendet wird, der eine selektive parallele Isolation mehrerer Spezies von Zielmolekülen, aus einem Gemisch erlaubt: Das Verfahren beinhaltet eine selektive Isolation von biochemisch funktionellen Zielmolekülen durch Bindung an ein Substrat mit immobilisierten Rezeptoren als Interaktionspartner. Die Funktionalität eines Zielmoleküls ist durch seine Fähigkeit zur selektiven Bindung an einen für das Zielmolekül spezifischen Rezeptor, vorzugsweise durch bioaffine Wechselwirkungen, wie Hybridisierung, Rezeptor-Ligand-Bindung definiert.

Das erfindungsgemäße Verfahren bedient sich dazu der Bereitstellung eines mikrofluidischen Trägers mit einem Array selektiv bindender Rezeptoren bzw. Fängersonden als Isolabonsmatrix für die Zielmoleküle. Es erfolgt eine in situ Synthese der Rezeptoren auf oder in dem mikrofluidischen. Reaktionsträger. Diese Vorgehensweise bei der Herstellung der Isolationsmatrix erlaubt bei Einsatz entsprechender Verfahren für die in situ Synthese einen sehr hohen Informationsgehalt der Isolationsmatrix. Im Fall von Nukleinsäuren als Zielmoleküle kann ein geeignetes System für die in situ Synthese der entsprechenden Fängersonden Tausende von definierten Sequenzen an der Isolationsmatrix herstellen. Somit wird durch die Erfindung ein Weg eröffnet, um gezielt Hunderte bis Tausende von individuellen DNA- oder RNA-Molekülen gezielt aus einem Gemisch zu extrahieren.

Der die immobilisierten Rezeptoren enthaltende Träger kann z.B. zum Einsatz kommen in der akademischen Forschung, der Grundlagenforschung, der industriellen Forschung, in der Qualitätskontrolle, in der Pharmaforschung, in der Biotechnologie, in der klinischen Forschung, in der klinischen Diagnostik, in Screening-Verfahren, in der patientenindividuellen Diagnostik, in klinischen Studien, in der Forensik, für genetische Tests, wie Eltemschaftsbestimmungen, in der Tier- und Pflanzenzucht oder im Umweltmonitoring.

Gegenstand der Erfindung ist somit ein Verfahren zur gezielten parallelen Extraktion von einer Vielzahl von individuellen DNA- oder RNA-Molekülen aus einem Gemisch, umfassend die Schritte
(a) Bereitstellen eines aus Hybridisierungssonden bestehenden Arrays auf oder in einem mikrofluidischen Träger als Isolationsmatrix, wobei die Hybridisierungssonden in situ schrittweise durch orts- oder/und zeitspezifisches Immobilisieren von Rezeptorbausteinen an den jeweils vorbestimmten Positionen auf dem oder im Träger aufgebaut werden und auf unterschiedlichen Positionen auf dem oder im Träger immobilisiert sind,
(b) Leiten einer Probe, die mehrere unterschiedliche DNA- und/oder RNA-Moleküle enthält, durch den mikrofluidischen Träger unter Bedingungen, bei denen die zu isolierenden DNA-und RNA-Moleküle spezifisch an die Isolationsmatrix binden,
(c) Entfernen von nicht an die Isolationsmatrix gebundenem Material und
(d) Eluieren der an die Isolationsmatrix gebunden unterschiedlichen DNA- oder RNA-Moleküle in einem einzigen Schritt.

Die durch das erfindungsgemäße Verfahren isolierten Zielmolekülen werden vorzugsweise aus Nukleinsäuren, z.B. doppelsträngigen oder einzelsträngigen DNA-Molekülen, z.B. genomischen DNA-Molekülen oder cDNA-Molekülen, oder RNA-Molekülen ausgewählt.

Bevorzugt handelt es sich bei den Zielmolekülen um Nukleinsäuren und die Rezeptoren auf dem mikrofluidischen Träger werden aus dazu komplementären Hybridisierungssonden ausgewählt. Bei den Hybridisierungssonden kann es sich ebenfalls um Nukleinsäuren, insbesondere DNA-Moleküle, jedoch auch um Nukleinsäureanaloga, wie Peptidnukleinsäuren (PNA), Locked-Nukleinsäuren (LNA) etc. handeln. Die Hybridisierungssonden haben vorzugsweise eine Länge entsprechend 10-100 Nukleotiden und müssen nicht durchgängig aus Bausteinen mit Basen bestehen, d.h. sie können beispielsweise auch abasische Bausteine, Linker, Spacer etc. enthalten. Die Hybridisierungssonden können am 3'-Ende, 5'-Ende oder dazwischen oder an mehreren Positionen am Träger gebunden sein.

Die Rezeptoren können auf dem mikrofluidischen Träger durch nichtkovalente oder kovalente Wechselwirkungen immobilisiert werden. Vorzugsweise erfolgt eine kovalente Immobilisierung, besonders bevorzugt über bi- oder polyfunktionelle Spacermoleküle. Alternativ ist eine Immobilisierung über elektrostatische Wechselwirkungen oder bioaffine Wechselwirkungen, z.B. Streptavidin/Biotin, Avidin/Biotin etc. bevorzugt.

Die auf dem Träger immobilisierten Rezeptoren, z.B. die Hybridisierungssonden, werden durch in situ Synthese, z.B. durch schrittweisen Aufbau aus Synthesebausteinen, auf dem Träger erzeugt und sind damit frei wählbar. Die Bausteine für diese in situ Synthese können Monomere der betreffenden Stoffklasse sein, also im Fall von Nukleinsäuren z.B. Nukleotidbausteine. Es kann sich aber auch um komplexere Bausteine handeln, so z.B. Oligonukleotide oder Oligopeptide aus mehreren, z.B. 2, 3, oder 4, Monomereinheiten.

Die für das erfindungsgemäße Verfahren verwendete Probe ist vorzugsweise eine komplexe Probe, d.h. die Zielmoleküle müssen aus einer Vielzahl ähnlicher Molekülspezies selektiv isoliert werden. Die Probe kann eine biologische Probe sein, z.B. eine Probe aus einem biologischen Organismus, z.B. aus einer Körperfiüssigkeit, eine Probe aus einer Zell- oder Mikroorganismenkultur etc. Weiterhin kann die Probe auch aus synthetischen Quellen, z.B. aus einer Synthesevorrichtung, stammen, oder ein Gemisch aus biologischem und synthetischem Material sein.

Die Probe kann vor dem Aufbringen auf den Träger gegebenenfalls prozessiert werden, z.B. durch enzymatische Reaktion, wie Amplifikation, Restriktionsspaltung, Markierung, Transkription, Translation, Fraktionierung, Vorreinigung etc. Die zu isolierenden Zielmoleküle können somit in markierter oder unmarkierter Form vorliegen.

Das erfindungsgemäße Verfahren wird bevorzugt an strukturierten Trägern, besonders bevorzugt an Trägern mit Kanälen, z.B. mit geschlossenen Kanälen, durchgeführt. Die Kanäle sind beispielsweise Mikrokanäle mit einem Querschnitt von 10-10 000 µm. Beispiele für geeignete Träger mit Kanälen sind in WO00/13017 und WO00/13018 beschrieben. Vorzugsweise werden Träger verwendet, die zumindest teilweise im Bereich der Positionen mit den immobilisierten Rezeptoren optisch transparent oder/ und elektrisch leitfähig sind.

Der für das erfindungsgemäße Verfahren eingesetzte mikrofluidische Träger enthält einen Array mit mehreren verschiedenen Rezeptoren, z.B. mit mindestens 10 und vorzugsweise mindestens 20 verschiedenen Rezeptoren. Die Rezeptoren werden vorzugsweise in situ schrittweise durch orts- oder/und zeitspezifisches Immobilisieren von Rezeptorbausteinen an den jeweils vorbestimmten Positionen auf dem oder im Träger aufgebaut.

An einer vorbestimmten Position kann nur eine einzige von Rezeptorspezies bzw. Rezeptorsequenz oder ein Gemisch von mehreren verschiedenen Rezeptorspezies bzw. Rezeptorsequenzen immobilisiert oder synthetisiert werden. Beispielsweise kann man Gemische von Rezeptorspezies verwenden, die für das gleiche zu extrahierende Zielmolekül spezifisch sind, z.B. ein Satz von verschiedenen Hybridisierungssonden für die Extraktion einer einzigen bestimmten Ziel-Nukleinsäure.

Das erfindungsgemäße Verfahren eignet sich insbesondere zum Einsatz als integriertes Synthese-Analyse(ISA)-Verfahren, d.h. ein Träger kann nach bestimmten Vorgaben in situ hergestellt und anschließend für einen Extraktionszyklus verwendet werden. Daran können sich gegebenenfalls weitere Synthese-Extraktions-Zyklen analog dem in WO00/13018 beschriebenen ISA-Prinzip anschließen. Auf diese Weise lassen sich mit einem Träger auch sehr unterschiedliche Moleküle, wie mRNA-Moleküle oder DNA-Sequenzen, extrahieren. Darüber hinaus ist eine Umstellung auf neue Extraktionsziele mit einem einzigen Träger möglich. Das Verfahren ist gut automatisierbar und kann mit weiteren nachfolgenden Prozessierungsschritten, wie einer Amplifikationsreaktion, z.B. unter Verwendung eines PCR-Thermocyclers, einer Detektion oder einer *in vitro* Translation kombiniert werden. Für diese nachfolgenden Prozessierungsschritte kann ein gemeinsamer, integrierter Reaktionsträger Anwendung finden.

Der Träger kann außer den eigentlichen Synthese/Reaktionsbereichen noch weitere mikrofluidische Funktionen bzw. Elemente beinhalten, wie etwa zusätzliche Reaktionsräume, z.B. für Enzymreaktionen innerhalb des Trägers, Reservoirs, Ventile, Pumpen, Thermoelemente, Reaktionsbereiche mit dort immobilisierten anderen biologisch-funktionalen Molekülen etc. Besonders bevorzugt sind Einrichtungen zur Temperierung des Trägers vorhanden, z.B. zum Einstellen einer bestimmten Temperatur oder eines bestimmten Temperaturprofils während des Extraktionsprozesses. Besonders bevorzugt enthält der Träger Thermoelemente, z.B. Peltier-Elemente, die eine orts- oder/und zeitspezifische Einstellung der Temperatur auf dem Träger oder einzelnen Bereichen des Trägers ermöglichen, für Hybridisierungen und nachfolgendes Aufschmelzen von Nukleinsäure-Doppelsträngen und für andere temperaturabhängige oder temperaturgesteuerte biochemische Reaktionen. Es können darüber hinaus auch Einrichtungen zur modulierten Zugabe von Puffern, z.B. aus unterschiedlichen Reservoirs, vorhanden sein, um Reaktionsbedingungen, z.B. Hybridisierungsbedingungen hinsichtlich der Stringenz, zeit- oder/und ortsspezifisch auf dem Träger oder Bereichen des Trägers zu modulieren.

Weiterhin kann der Träger zusätzliche funktionale biologische Moleküle in immobilisierter Form enthalten, beispielsweise Polymersonden, die zur Analyse der isolierten Zielmoleküle, z.B. zur Qualitätskontrolle, dienen. Weiterhin können funktionale biologische Moleküle in immobilisierter Form für enzymatische Reaktionen, z.B. Enzyme, PCR-Primer, Ribozyme etc., Modifikationen oder Derivatisierungen der Zielmoleküle vorliegen.

In einer weiteren Ausführungsform ist der Träger in einer Vorrichtung integriert, umfassend eine programmierbare Lichtquellenmatrix, eine Detektormatrix, einen vorzugsweise zwischen Lichtquellen- und Detektormatrix angeordneten Träger sowie Mittel zur Zufuhr von Fluids in den Träger und zur Ableitung von Fluids aus dem Träger. Die programmierbare Lichtquellen- bzw. Belichtungsmatrix kann eine Reflexionsmatrix, eine Lichtventilmatrix, z.B. eine LCD-Matrix oder eine selbstemittierende Belichtungsmatrix, sein. Derartige Lichtmatrices sind in WO00/13017 und WO00/13018 offenbart. Die Detektormatrix kann gegebenenfalls im Trägerkörper integriert sein.

Der in situ Aufbau von Rezeptoren auf dem Träger kann fluidchemische Schritte, photochemische Schritte, elektrochemische Schritte oder Kombinationen von zwei oder mehreren dieser Schritte umfassen. Ein Beispiel für eine elektrochemische Synthese von Rezeptoren auf einem Träger ist in DE 101 20 633.1 beschrieben. Ein Beispiel für ein Hybridverfahren, umfassend die Kombination von fluidchemischen Schritten und photochemischen Schritten, ist in DE 101 22 357.9 beschrieben.

Die Verwendung eines Trägers, umfassend einen Array mit einer Vielzahl verschiedener Rezeptoren, ermöglicht die Immobilisierung von mehreren unterschiedlichen Zielmolekülen aus einer komplexen Probe in einem einzigen Schritt. Die Elution der auf dem Träger immobilisierten Zielmoleküle erfolgt in einem einzigen Schritt. Bevorzugt ist eine orts- oder/und zeitspezifische Elution, wobei einzelne Zielmolekülen oder einzelne Gruppen von Zielmolekülen zunächst in einem ersten Schritt von Träger eluiert werden und die Elution weiterer Zielmoleküle oder Gruppen von Zielmolekülen in einem oder mehreren nachfolgenden Schritten erfolgt. Diese orts- oder/und zeitspezifische Elution umfasst vorzugsweise fluidchemische, photochemische oder elektrochemische Schritte oder Kombinationen davon. Ebenfalls bevorzugt ist die Verwendung von lokalen Temperaturänderungen auf dem Träger.

In einer besonders bevorzugten Ausführungsform der Erfindung erfolgt die Elution mittels einer ortsspezifischen Temperaturänderung, z.B. einer Temperaturerhöhung, die zu einer Denaturierung von Nukleinsäure-Doppelsträngen führt. Eine derartige ortsspezifische Temperaturänderung kann durch ortsspezifische Thermoelemente oder/und ortsaufgelöste Einwirkung von Strahlung, z.B. UV/VIS- und insbesondere IR-Strahlung, erfolgen, die ortsaufgelöst zu einer Wärmeentwicklung führt.

Eine separate orts- oder/und zeitspezifische Elution unterschiedlicher Zielmoleküle kann gegebenenfalls auch durch Abtrennung einzelner Reaktionsräume mittels hydrophober Barrieren entlang von Kanälen erreicht werden, um die diffusionsbedingten Vermischungen von eluierten Zielmolekülen (Nukleinsäuren) zu vermeiden.

Durch orts- oder/und zeitspezifische Elution können auch gezielt Gemische von ausgewählten Zielmolekülen erzeugt werden, wobei z.B. mehrere Fraktionen von Zielmolekülgemischen erhalten werden. Desweiteren können Zielmolekül-Gradienten bzw. Gradientenmischungen durch unterschiedliche Elutionseinwirkung an verschiedenen Stellplätzen, z.B. durch unterschiedlich lange Temperatureinwirkungen, hergestellt werden.

Das erfindungsgemäße Verfahren hat gegenüber dem Stand der Technik den Vorteil, dass es eine selektive Isolation von mehreren Zielmolekülen durch einen einzigen Arbeitsvorgang erlaubt. Die Verwendung mikrofluidischer Träger bewirkt, dass hierzu mit geringen Probenvolumina von z.B. 0,1 µl bis 100 µl gearbeitet werden kann. Trotz dieser geringen Probenvolumina werden im Träger hohe lokale Konzentrationen der Zielmoleküle erreicht, so dass sehr spezifisch und umfassend extrahiert werden kann.

Durch das mikrofluidische Format wird bei der Extraktion keine Aerosolbildung und dadurch Querkontamination hervorgerufen, wie es z.B. bei säulengestützten Verfahren mit Zentrifugationsschritt oder Vakuumabsaugung der Fall ist. Außerdem findet keine Kompetition von unterschiedlich häufigen oder/und in einer Tertiärstruktur vorliegenden Nukleinsäuren um einheitliche Bindungsstellen statt, welches ansonsten zum Vorteil der häufigeren bzw. weniger gefalteten Moleküle verlaufen kann, wie dies bei poly-A Säulen der Fall ist. Die durch das erfindungsgemäße Verfahren selektiv extrahierten Nukleinsäuren sind daher in anschließenden Schritten, wie z.B. PCR, leichter zu handhaben als ein Gemisch, wodurch weniger Probleme, z.B. falsche Bindung von Primern etc., hervorgerufen werden. Schließlich kann das erfindungsgemäße Verfahren auch leicht automatisiert werden. Insbesondere können vorher oder nachher stattfindende Prozesse automatisch angekoppelt werden, z.B. eine enzymatische Reaktion oder eine Ablage der isolierten Moleküle in geeigneten Behältern.

Ein zusätzlicher Vorteil der Verwendung mikrofluidischer Träger als Isolationsmatrix besteht darin, dass ein oder mehrere Zyklen der Bindung und anschließenden Elution von Zielmolekülen nach dem Durchflussprinzip durchgeführt werden können, wobei ein derartiger Zyklus die Schritte: Binden, Waschen unerwünschter Moleküle, Eluieren der gewünschten Moleküle und Rekonstituieren des Trägers umfasst. Die Isolation kann auch unter Zirkulation des Zielmolekülgemisches zur Verbesserung von aktiven Wechselwirkungen zwischen Rezeptor und Analyt bzw. Verbesserung des Waschvorgangs und der Rekonstitution erfolgen.

Die extrahierten Zielmoleküle können direkt oder indirekt für diagnostische oder therapeutische Zwecke eingesetzt werden. Weiterhin kann das extrahierte Material in Nachfolgereaktionen eingesetzt werden. So können aus extrahierten Nukleinsäuren Proteine oder Peptide, z.B. durch Transfer in geeignete Vektoren (Klonierung), oder in geeignete Zielzellen (Transformation bzw. Transfektion) oder durch *in vitro* Translation, insbesondere Isolierung von mRNA-Zielmolekülen, erzeugt werden.

Im Folgenden ist der schematische Ablauf des Verfahrens am Beispiel von Nukleinsäuren als Zielmolekülen erläutert:
1. Ermittlung von Daten zu den Sequenzen der Zielmoleküle, die aus einer Probe extrahiert werden sollen, z.B. 20 Gene aus einem Modellorganismus, der gerade untersucht wird; mit zumindest teilweise bekannten Sequenzen der 20 Gene;
2. Ermittlung von geeigneten Fängersonden, die komplementär zu singulären Bereichen in den ausgewählten Genen sind, bevorzugt mit Hilfe eines Computer-Algorithmus;
3. Vorbereitung der Probe durch Isolation von Gesamt-RNA aus Zellen des Modellorganismus;
4. Bereitstellen eines Geräts zur in situ Synthese von entsprechenden Fängersonden in oder auf einem mikrofluidischen Reaktionsträger;
5. Einspeisen der ermittelten Fängersonden-Sequenz in eine Synthese-Steuerungseinheit, die mit dem mikrofluidischen Träger integriert sein kann;
6. Synthetisieren der ausgewählten Fängersonden-Sequenz in oder auf dem mikrofluidischen Reaktionsträger;
7. Zugeben der Probe, so dass die Probe in die Kanäle des Reaktionsträgers gespült wird;
8. Equilibrieren der Bedingungen auf dem Träger, so dass eine Bindung zwischen Zielmolekülen und Fängersonden (Hybridisierung) bei geeigneten Pufferbedingungen und geeigneter Temperatur erfolgen kann;
9. Spülen der Kanäle, um ungebundenes Material zu entfernen;
10. Wechseln des Puffers (Stringenz) und eventuell der Temperatur, um die spezifischen Hybride zwischen Zielmolekülen und Fängersonden zu zerstören;
11. Gezieltes Ausspülen und Auffangen der abgelösten Zielmoleküle;
12. Weitere Verwendung der selektiv extrahierten Nukleinsäuren, z.B. in PCR und Klonierung, um dann nach Expression die Wirkung der Genprodukte zu studieren.

Das oben beschriebene Prinzip kann auch in abgewandelter Form zur selektiven Entfernung von bestimmten Komponenten (vorzugsweise mehr als eine Spezies) aus einer ein Zielmolekül oder ein Zielmolekül-Gemisch enthaltenden Probe verwendet werden. Störende Komponenten können entfernt werden, z.B. repetitive Elemente oder Telomersequenzen bei Analyse von Genfragmenten; Herausfiltern von bestimmten Genen (z.B. Housekeeping Genes) bei Transkriptionsanalysen. So können bekannte Komponenten abgefangen und nur noch erwünschte, z.B. bekannte oder unbekannte, Zielmoleküle eluiert werden. Das führt zu einer Aufkonzentration erwünschter Nukleinsäuren oder Proteine oder anderer erwünschter Biomoleküle.

Der Ablauf des Verfahrens bei einer derartigen Ausführungsform ist wie folgt:
1. Ermittlung von Daten zu den Sequenzen störender Komponenten;
2. Ermittlung geeigneter Fängersonden für störende Komponenten;
3-7. wie oben;
8. Equilibrieren bei Bedingungen, bei denen störende Komponenten binden;
9. Gezieltes Ausspülen und Auffangen der gewünschten Moleküle (störende Elemente verbleiben auf dem Träger).

Durch diese Vorgehensweise werden störende Komponenten abgereichert, so dass nachfolgende Analyse der gewünschten Moleküle mit höherer Präzision durchgeführt werden können. Weiterhin wird eine Aufkonzentrierung der gewünschten Zielmoleküle der zu untersuchenden Probe erreicht und störende Moleküle, z.B. mehrere Spezies parallel, können gezielt aus dem Molekülgemisch entfernt werden.

Es wird somit ein Verfahren zur Isolierung von Zielmolekülen aus einer Probe beschreiben umfassend die Schritte
(a) Bereitstellen eines mikrofluidischen Trägers mit einem Array von mehreren unterschiedlichen Rezeptoren, die auf jeweils unterschiedlichen Positionen auf dem oder im Träger immobilisiert sind,
(b) Leiten einer Probe, die zu isolierende Zielmoleküle enthält, durch den mikrofluidischen Träger, unter Bedingungen, bei denen störende Moleküle spezifisch an die auf dem Träger immobilisierten Rezeptoren binden können, und
(c) Eluieren der nichtgebundenen Zielmoleküle vom Träger.

Die Verfahrensführung bei dieser Ausführungsform erfolgt entsprechend den Erläuterungen für die erste Ausführungsform, mit der Maßgabe, dass die in der Probe vorhandenen Zielmoleküle nicht oder in geringerem Umfang als die störenden Komponenten an die Rezeptoren auf dem Träger binden.

Die vorliegende Erfindung umfasst schließlich auch eine Ausführungsform betreffend ein Verfahren zur Isolierung von Zielmolekülen aus einer Probe, wobei mindestens ein Verfahrenszyklus, bei dem die Zielmoleküle an die Rezeptoren binden und mindestens einen Verfahrenszyklus, bei dem die Zielmolekülen nicht oder nur gering an die Rezeptoren binden, kombiniert werden. Vorzugsweise wird zunächst ein Verfahrenszyklus durchgeführt, bei dem die Zielmoleküle nicht oder nur gering binden, an den sich ein Verfahrensryklus anschließt, bei dem die Zielmoleküle binden und dann ort- oder/und zeitspezifisch vom Träger eluiert werden.

Weiterhin soll die Erfindung durch die folgenden Abbildungen näher erläutert werden.

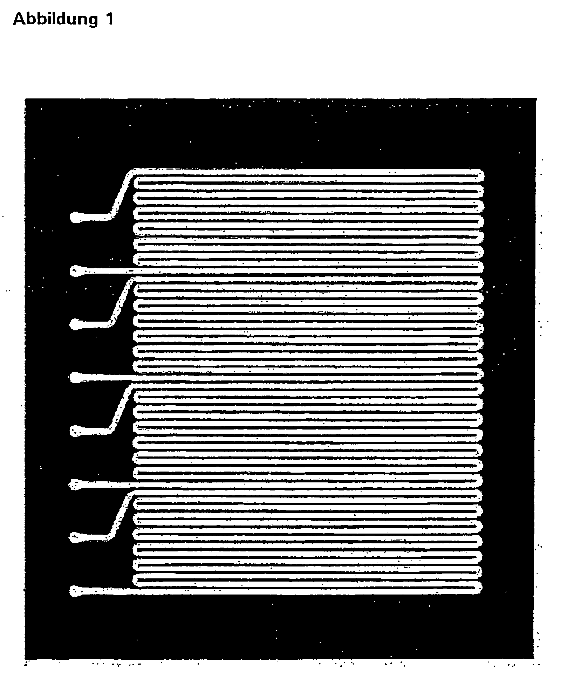

Abbildung 1 zeigt ein Beispiel für einen geeigneten mikrofluidischen Reaktionsträger mit insgesamt 4 separaten Kanälen.

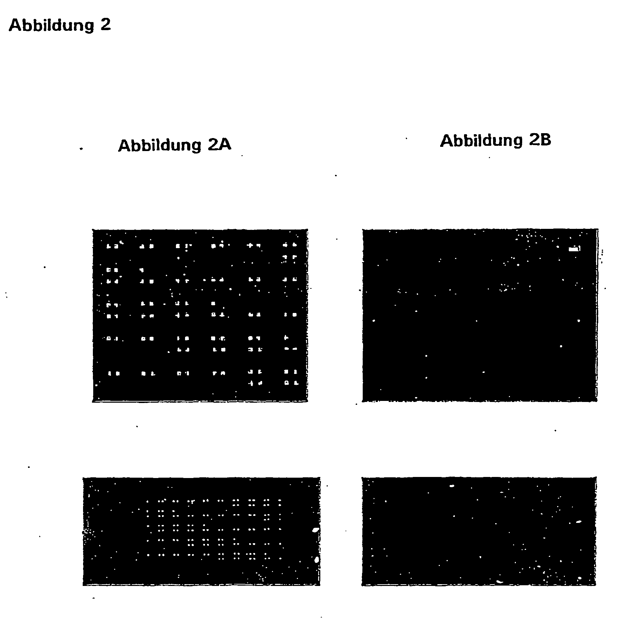

Abbildung 2 zeigt die spezifische Hybridisierung von Nukleinsäure-Zielmolekülen an einem mikrofluidischen Reaktionsträger und deren quantitative Extrahierbarkeit. In Abbildung 2A ist die Hybridisierung eines Zielmoleküls an vorbestimmten Posititonen auf einem mikrofluidischen Reaktionsträger erkennbar. Abbildung 2B zeigt den Träger nach Extraktion. Es ist zu erkennen, dass eine quantitative Elution des Zielmoleküls erfolgt ist.

Folgende Gegenstände werden ebenfalls beschrieben:
1. Verfahren zur Isolierung von Zielmolekülen aus einer Probe, umfassend die Schritte:
   (a) Bereitstellen eines mikrofluidischen Trägers mit einem Array von mehreren verschiedenen Rezeptoren, die auf jeweils unterschiedlichen Positionen auf dem oder im Träger immobilisiert sind,
   (b) Leiten einer Probe, die zu isolierende Zielmoleküle enthält, durch den mikrofluidischen Träger, unter Bedingungen, bei denen Zielmoleküle spezifisch an die auf dem Träger immobilisierten Rezeptoren binden können,
   (c) Entfernen von nichtgebundenem Material aus der Probe vom Träger und
   (d) Eluieren der an den Träger gebundenen Zielmoleküle.
2. Verfahren nach Punkt 1,
   dadurch gekennzeichnet,
   dass die Zielmoleküle ausgewählt werden aus Nukleinsäuren, Polypeptiden, Peptiden und Sacchariden.
3. Verfahren nach Punkt 1 oder 2,
   dadurch gekennzeichnet,
   dass die Zielmoleküle aus Nukleinsäuren, insbesondere DNA- oder/und RNA-Molekülen ausgewählt werden.
4. Verfahren nach Punkt 3,
   dadurch gekennzeichnet,
   dass als Rezeptoren Hybridisierungssonden verwendet werden.
5. Verfahren nach Punkt 4,
   dadurch gekennzeichnet,
   dass man als Hybridisierungssonden Nukleinsäuren oder Nukleinsäureanaloga verwendet.
6. Verfahren nach Punkt 4 oder 5,
   dadurch gekennzeichnet,
   dass die Hybridisierungssonden eine Länge entsprechend 10-100 Nukleotiden aufweisen.
7. Verfahren nach einem der vorhergehenden Punkte,
   dadurch gekennzeichnet,
   dass man eine Probe biologischen oder/und synthetischen Ursprungs verwendet.
8. Verfahren nach einem der vorhergehenden Punkte,
   dadurch gekennzeichnet,
   dass man eine Probe verwendet, die einem oder mehreren Vorbehandlungsschritten unterzogen worden ist.
9. Verfahren nach einem der vorhergehenden Punkte,
   dadurch gekennzeichnet,
   dass man einen Träger mit geschlossenen Kanälen verwendet.
10. Verfahren nach Punkt 9,
   dadurch gekennzeichnet,
   dass der Träger Mikrokanäle mit einem Durchmesser von 10-1000 µm aufweist.
11. Verfahren nach einem der vorhergehenden Punkte,
   dadurch gekennzeichnet,
   dass der Array auf dem Träger mindestens 10, vorzugsweise mindestens 20 Positionen mit verschiedenen Rezeptoren enthält.
12. Verfahren nach einem der vorhergehenden Punkte,
   dadurch gekennzeichnet,
   dass die Rezeptoren an den einzelnen Positionen Einzelsequenzen oder/und Sequenzgemische enthalten.
13. Verfahren nach einem der vorhergehenden Punkte,
   dadurch gekennzeichnet,
   dass die Rezeptoren des Arrays in situ schrittweise durch orts- oder/und zeitspezifisches Immobilisieren von Rezeptorbausteinen an den jeweils vorbestimmten Positionen auf dem oder im Träger aufgebaut werden.
14. Verfahren nach Punkt 13,
   dadurch gekennzeichnet,
   dass man den Träger für einen oder mehrere integrierte Synthese-Analyse-Zyklen einsetzt.
15. Verfahren nach Punkt 13 oder 14,
   dadurch gekennzeichnet,
   dass man den Träger zusammen mit einer programmierbaren Lichtquellenmatrix und einer Detektionsmatrix verwendet.
16. Verfahren nach einem der vorhergehenden Punkte,
   dadurch gekennzeichnet,
   dass die an den Träger gebundenen Zielmoleküle orts- oder und zeitspezifisch eluiert werden.
17. Verfahren nach Punkt 16,
   dadurch gekennzeichnet,
   dass die orts- oder/und zeitspezifische Elution fluidchemische, photochemische oder elektrochemische Schritte oder Kombinationen davon umfasst.
18. Verfahren nach Punkt 17,
   dadurch gekennzeichnet,
   dass man einen Träger mit lichtempfindlichen Rezeptoren verwendet.
19. Verfahren nach Punkt 16,
   dadurch gekennzeichnet,
   dass die orts- oder/und zeitspezifische Elution eine orts- oder/und zeitspezifische Temperaturänderung auf dem Träger umfasst.
20. Verfahren nach Punkt 19,
   dadurch gekennzeichnet,
   dass man einen Träger mit integrierten Thermoelementen verwendet.
21. Verfahren nach einem der vorhergehenden Punkte,
   dadurch gekennzeichnet,
   dass man die extrahierten Zielmoleküle einer Nachfolgereaktion unterzieht.
22. Verfahren nach einem der vorhergehenden Punkte,
   dadurch gekennzeichnet,
   dass man die extrahierten Zielmoleküle direkt oder indirekt für diagnostische oder therapeutische Zwecke einsetzt.
23. Verfahren nach einem der vorhergehenden Punkte,
   dadurch gekennzeichnet,
   dass ein Träger verwendet wird, der zusätzliche funktionale biologische Moleküle in immobilisierter Form enthält.
24. Verfahren nach Punkt,
   dadurch gekennzeichnet,
   dass die zusätzlichen funktionalen biologischen Moleküle Polymersonden sind und der Analyse der isolierten Zielmoleküle dienen.
25. Verfahren zur Isolierung von Zielmolekülen aus einer Probe, umfassend die Schritte:
   (a) Bereitstellen eines mikrofluidischen Trägers mit einem Array von mehreren verschiedenen Rezeptoren, die auf jeweils unterschiedlichen Positionen auf dem oder im Träger immobilisiert sind,
   (b) Leiten einer Probe, die zu isolierende Zielmoleküle enthält, durch den mikrofluidischen Träger, unter Bedingungen, bei denen störende Komponenten aus einer Probe spezifisch an die auf dem Träger immobilisierten Rezeptoren binden können, und
   (c) Eluieren der nichtgebundenen Zielmoleküle vom Träger.
26. Verfahren zur Isolierung von Zielmolekülen aus einer Probe, umfassend mindestens einen Verfahrenszyklus nach Anspruch 1 und mindestens einen Verfahrenszyklus nach Punkt 25.

## Patentansprüche

1. Verfahren zur gezielten parallelen Extraktion von einer Vielzahl von individuellen DNA- oder RNA-Molekülen aus einem Gemisch, umfassend die Schritte
(a) Bereitstellen eines aus Hybridisierungssonden bestehenden Arrays auf oder in einem mikrofluidischen Träger als Isolationsmatrix, wobei die Hybridisierungssonden in situ schrittweise durch orts- oder/und zeitspezifisches Immobilisieren von Rezeptorbausteinen an den jeweils vorbestimmten Positionen auf dem oder im Träger aufgebaut werden und auf unterschiedlichen Positionen auf dem oder im Träger immobilisiert sind,
(b) Leiten einer Probe, die mehrere unterschiedliche DNA- und/oder RNA-Moleküle enthält, durch den mikrofluidischen Träger unter Bedingungen, bei denen die zu isolierenden DNA-und RNA-Moleküle spezifisch an die Isolationsmatrix binden,
(c) Entfernen von nicht an die Isolationsmatrix gebundenem Material und
(d) Eluieren der an die Isolationsmatrix gebunden unterschiedlichen DNA- oder RNA-Moleküle in einem einzigen Schritt.

2. Verfahren nach Anspruch 1, **dadurch gekennzeichnet, dass** die Hybridisierungssonden komplementär zu den zu extrahierenden DNA- oder RNA-Molekülen sind.

3. Verfahren nach Anspruch 1, **dadurch gekennzeichnet, dass** sich weitere Synthese- Extraktionszyklen anschließen.

4. Verfahren nach Anspruch 1, **dadurch gekennzeichnet, dass** sich eine Rekonstitution des Trägers anschließt.

5. Verfahren nach Anspruch 1, **dadurch gekennzeichnet, dass** die extrahierten Zielmoleküle in einer oder mehreren Nachfolgereaktionen eingesetzt werden.

6. Verfahren nach Anspruch 5, **dadurch gekennzeichnet, dass** es sich bei der Nachfolgereaktion um eine Amplifikation, PCR, eine Klonierung, Transfektion, Translation oder um eine *in vitro*-Translation handelt.

7. Verfahren nach Anspruch 1, **dadurch gekennzeichnet, dass** in Schritt (b) die Bindung der Zielmoleküle unter Zirkulation des Zielmolekülgemisches erfolgt.

8. Verfahren nach Anspruch 1, **dadurch gekennzeichnet, dass** die Ermittlung der geeigneten Hybridisierungssonden mit Hilfe eines Computeralgorithmus erfolgt und diese komplementär zu den Zielmolekülen sind.

9. Verfahren nach Anspruch 1, **dadurch gekennzeichnet, dass** mehr als eine Hybridisierungssonde für die Extraktion einer einzigen bestimmten Ziel-Nukleinsäure verwendet wird.

10. Verfahren zur selektiven Extraktion von Nukleinsäuren-Zielmolekülen nach Anspruch 1, umfassend
(a) Ermitteln von Daten zu den Sequenzen der Zielmoleküle, die aus einer Probe extrahiert werden sollen,
(b) Ermitteln von geeigneten Hybridisierungssonden,
(c) Bereitstellen eines Gerätes zur *in situ*-Synthese von Hybridisierungssonden auf einem mikrofluidischen Reaktionsträger,
(d) Einspeisen der Hybridisierungssondensequenzen in eine Synthese-Steuerungseinheit,
(e) Synthetisieren der Hybridisierungssondensequenzen in oder auf dem mikrofluidischen Reaktionsträger,
(f) Zugeben der Probe, aus der die Zielmoleküle extrahiert werden sollen, so dass die Probe in die Kanäle des Reaktionsträgers gespült wird,
(g) Equilibrieren des Träger so dass eine Bindung zwischen Zielmolekülen und Hybridisierungssonden erfolgen kann,
(h) Spülen des Trägers, um ungebundenes Material zu entfernen,
(i) Zerstören der Hybride zwischen Zielmolekülen und Hybridisierungssonden,
(j) Ausspülen und Auffangen der abgelösten Zielmoleküle und
(k) weiteres Verwenden der selektiv extrahierten Nukleinsäuren.

11. Verfahren nach Anspruch 1, **dadurch gekennzeichnet, dass** die Elution gemäß Schritt (d) eine orts- oder/und zeitspezifische Elution ist.

12. Verfahren nach Anspruch 11, wobei durch die Elution Gemische von ausgewählten Zielmolekülen erzeugt werden.

13. Verfahren nach Anspruch 11, wobei durch die Elution einzelne Zielmoleküle oder einzelne Gruppen von Zielmolekülen zunächst in einem ersten Schritt vom Träger eluiert werden und die Elution weiterer Zielmoleküle oder Gruppen von Zielmolekülen in einem oder mehreren nachfolgenden Schritten erfolgt.

14. Verfahren gemäß Anspruch 1, **dadurch gekennzeichnet, dass** die Hybridisierungssonden an den einzelnen Positionen Einzelsequenzen oder/und Sequenzgemische enthalten.

15. Verfahren nach einem der Ansprüche 1-14, **dadurch gekennzeichnet, dass** die Synthese der Hybridisierungssonden *in situ* mit fluidchemischen Schritten, photochemischen Schritten, elektrochemischen Schritten oder Kombinationen von zwei oder mehreren dieser Schritte erfolgt.

## Claims

1. Method for the targeted parallel extraction of a plurality of individual DNA or RNA molecules from a mixture comprising the steps
(a) providing an array consisting of hybridization probes on or in a microfluidic carrier as an isolation matrix, wherein the hybridization probes are synthesized stepwise in situ by site-specific or/and time-specific immobilization of receptor building blocks at the respectively predetermined positions on or in the carrier and are immobilized at different positions on or in the carrier,
(b) passing a sample which contains several different DNA and/or RNA molecules through the microfluidic carrier under conditions in which the DNA and RNA molecules to be isolated can specifically bind to the isolation matrix,
(c) removing material that has not bound to the isolation matrix and
(d) eluting the different DNA or RNA molecules that have bound to the isolation matrix in a single step.

2. Method according to claim 1, **characterized in that** the hybridization probes are complementary to the DNA or RNA molecules to be extracted.

3. Method according to claim 1, **characterized in that** it is followed by additional synthesis - extraction cycles.

4. Method according to claim 1, **characterized in that** it is followed by a reconstitution of the carrier.

5. Method according to claim 1, **characterized in that** the extracted target molecules are used in one or more subsequent reactions.

6. Method according to claim 5, **characterized in that** the subsequent reaction is an amplification, PCR, a cloning, transfection, translation or an *in vitro-*translation.

7. Method according to claim 1, **characterized in that** in step (b) the binding of the target molecules takes place while the target molecule mixture is circulated.

8. Method according to claim 1, **characterized in that** suitable hybridization probes are determined with the aid of a computer algorithm and they are complementary to the target molecules.

9. Method according to claim 1, **characterized in that** more than one hybridization probe is used to extract a single particular target nucleic acid.

10. Method for the selective extraction of nucleic acid target molecules according to claim 1, comprising
(a) determining data on the sequences of the target molecules which are to be extracted from a sample,
(b) determining suitable hybridization probes,
(c) providing a device for the in situ synthesis of hybridization probes on a microfluidic reaction carrier,
(d) feeding in the hybridization probe sequences into a synthesis control unit,
(e) synthesizing the hybridization probe sequences in or on the microfluidic reaction carrier,
(f) adding the sample from which the target molecules are to be extracted such that the sample is flushed into the channels of the reaction carrier,
(g) equilibrating the carrier such that a binding can take place between target molecules and hybridization probes,
(h) flushing the carrier in order to remove unbound material,
(i) destroying the hybrids between target molecules and hybridization probes,
(j) rinsing out and capturing the detached target molecules and
(k) further use of the selectively extracted nucleic acids.

11. Method according to claim 1, **characterized in that** the elution according to step (d) is a site-specific or/and time-specific elution.

12. Method according to claim 11, wherein mixtures of selected target molecules are produced by the elution.

13. Method according to claim 11, wherein individual target molecules or individual groups of target molecules are firstly eluted from the carrier in a first step by the elution and further target molecules or groups of target molecules are eluted in one or more subsequent steps.

14. Method according to claim 1, **characterized in that** the hybridization probes contain single sequences or/and sequence mixtures at the individual positions.

15. Method according to one of the claims 1 - 14, **characterized in that** the synthesis of the hybridization probes in situ is carried out with fluid-chemical steps, photochemical steps, electrochemical steps or combinations of two or more of these steps.

## Revendications

1. Procédé d'extraction parallèle ciblée d'une multiplicité de molécules d'ADN ou d'ARN individuelles à partir d'un mélange, comprenant les étapes consistant à :
(a) se munir d'une puce consistant en sondes d'hybridation sur ou dans un support microfluidique comme matrice d'isolement, dans lequel les sondes d'hybridation sont élaborées *in situ* par étapes par immobilisation spécifique au site et/ou au temps d'éléments constitutifs de récepteur sur les positions prédéterminées respectives sur ou dans le support et sont immobilisées sur des positions différentes sur ou dans le support,
(b) faire passer un échantillon qui contient plusieurs molécules différentes d'ADN et/ou d'ARN à travers le support microfluidique dans des conditions dans lesquelles les molécules d'ADN et d'ARN à isoler peuvent se lier spécifiquement à la matrice d'isolement,
(c) éliminer le matériau non lié à la matrice d'isolement et
(d) procéder à une élution des différentes molécules d'ADN ou d'ARN liées à la matrice d'isolement dans une étape unique.

2. Procédé selon la revendication 1, **caractérisé en ce que** les sondes d'hybridation sont complémentaires aux molécules d'ADN ou d'ARN à extraire.

3. Procédé selon la revendication 1, **caractérisé en ce que** l'on procède à d'autres cycles de synthèse-extraction.

4. Procédé selon la revendication 1, **caractérisé en ce que** l'on procède à une reconstitution du support.

5. Procédé selon la revendication 1, **caractérisé en ce que** les molécules cibles extraites sont utilisées dans une ou plusieurs réactions consécutives.

6. Procédé selon la revendication 5, **caractérisé en ce que** la réaction consécutive est une amplification, une PCR, un clonage, une transfection, une traduction ou une traduction *in vitro.*

7. Procédé selon la revendication 1, **caractérisé en ce qu'**à l'étape b, la liaison des molécules cibles s'effectue par circulation du mélange de molécules cibles.

8. Procédé selon la revendication 1, **caractérisé en ce que** la détermination des sondes d'hybridation appropriées s'effectue à l'aide d'un algorithme informatique et celles-ci sont complémentaires aux molécules cibles.

9. Procédé selon la revendication 1, **caractérisé en ce que** plusieurs sondes d'hybridation sont utilisées pour l'extraction d'un acide nucléique cible déterminé.

10. Procédé d'extraction sélective de molécules cibles d'acides nucléiques selon la revendication 1, comprenant
(a) la détermination de données sur les séquences des molécules cibles qui doivent être extraites d'une sonde,
(b) la détermination de sondes d'hybridation appropriées
(c) la mise à disposition d'un appareil de synthèse *in situ* de sondes d'hybridation sur un support réactionnel microfluidique,
(d) l'apport des séquences de sondes d'hybridation dans une unité de commande de la synthèse,
(e) la synthèse des séquences de sondes d'hybridation dans ou sur le support réactionnel microfluidique,
(f) l'addition de la sonde de laquelle les molécules cibles doivent être extraites de sorte que la sonde soit rincée dans les canaux du support réactionnel,
(g) l'équilibrage du support de sorte qu'une liaison entre les molécules cibles et les sondes d'hybridation puisse se produire,
(h) le rinçage du support pour éliminer le matériau non lié,
(i) la destruction des hybrides entre les molécules cibles et les sondes d'hybridation,
(j) le rinçage et la capture des molécules cibles détachées et
(k) l'utilisation ultérieure des acides nucléiques extraits sélectivement.

11. Procédé selon la revendication 1, **caractérisé en ce que** l'élution est réalisée selon l'étape (d) de façon spécifique au site et/ou au temps.

12. Procédé selon la revendication 11, dans lequel des mélanges de molécules cibles choisies sont générés par l'élution.

13. Procédé selon la revendication 11, dans lequel, par élution, des molécules cibles individuelles ou des groupes individuels de molécules cibles sont tout d'abord élués du support dans une première étape et l'élution d'autres molécules cibles ou groupes de molécules cibles s'effectue dans une ou plusieurs étapes consécutives.

14. Procédé selon la revendication 1, **caractérisé en ce que** les sondes d'hybridation contiennent sur les positions individuelles, des séquences individuelles et/ou des mélanges de séquences.

15. Procédé selon l'une des revendications 1 à 14, **caractérisé en ce que** la synthèse des sondes d'hybridation s'effectue *in situ* par des étapes de chimie fluide, des étapes photochimiques, des étapes électrochimiques ou des combinaisons de deux ou plus de ces étapes.
